# EUROPÄISCHE PATENTSCHRIFT

(11) **EP 0 684 302 B1**
(45) Veröffentlichungstag und Bekanntmachung des Hinweises auf die Patenterteilung: **24.11.1999**
(21) Anmeldenummer: 95107293.3
(22) Anmeldetag: 13.05.1995
(51) Int. Cl.: C11D 1/66, C11D 3/20, A61K 7/08

(54) **Wässrige, fliessfähige Perlglanzkonzentrate**
Aqueous free-flowing lustre concentrates
Produits concentrés à lustre perlaire coulants et aqueux

(30) Priorität: 28.05.1994 DE 4418718
(43) Veröffentlichungstag der Anmeldung: 29.11.1995
(73) Patentinhaber: Th. Goldschmidt AG, 45127 Essen (DE)
(72) Erfinder: Leidreiter, Holger, Dr., D-45529 Hattingen (DE)

(56) Entgegenhaltungen:
- EP-A- 0 569 028
- EP-A- 0 586 323
- DE-A- 4 103 551
- US-A- 4 343 726

## Beschreibung

Die Erfindung betrifft wäßrige, fließfähige Perlglanzkonzentrate, welche
a) 10 - 40 % perlglanzgebende Komponenten,
b) 0 - 50 % Dispergierhilfsmittel und
c) 0,2 - 20 % Kristallisationshilfsmittel
enthalten.

Kosmetische Haar- und Körperreinigungsmittel, Geschirrspülmittel und flüssige Wasch- und Reinigungsmittel enthalten zur Verbesserung des optischen Aspektes, und damit zur Steigerung des Handelswertes, häufig Substanzen, die den genannten Präparaten ein perlglanzartiges Aussehen verleihen. Um einen solchen Perlglanz- bzw. Seidenglanz-Effekt zu erreichen, sind verschiedene Substanzen bekannt, beispielsweise pulverförmige Naturstoffe, wie Glimmer, Fischsilber, anorganische Materialien, wie Wismutoxichlorid und Titandioxidpigmente, ferner Metallsalze höherer Fettsäuren, Fettsäureglykolester und Fettsäurealkanolamide, gegebenenfalls im Gemisch mit anderen Tensiden.

Üblicherweise werden zu diesem Zweck wäßrige, fließfähige Perlglanzkonzentrate verwendet, welche z. B. organische perlglanzgebende Substanzen enthalten, die dann kalt in Tensidlösungen eingebracht werden. Diese Perlglanzkonzentrate können vom Stand der Technik ausgehend wie folgt zusammengesetzt sein:
1. aus einer perlglanzgebenden Komponente, wie z. B. Fettsäureglykolester oder Fettsäureglycerinester der Stearinsäure,
2. aus Dispergiermitteln, wie z. B. anionischen Tensiden (Laurylethersulfat) oder Fettsäureamidopropylbetainen,
3. aus Kristallisationshilfsmitteln, die den Zweck haben, die perlglanzgebende Komponente in der gewünschten Form kristallisieren zu lassen.

Die Kristallisation wird durch das rheologische Verhalten der Mischung und die vom Dispergiermittel und dem Kristallisationshilfsmittel gebildete Micellstruktur bzw. Emulsionsstruktur beeinflußt.

Aus dem Stand der Technik sind Cocosfettsäureethanolamide als Kristallisationshilfsmittel bekannt, bei denen aber die Gefahr besteht, daß aufgrund vorhergehender Restmengen an sekundären Aminen gesundheitsgefährdende Nitrosamine gebildet werden können. Weiterhin ist aus der DE-A-42 13 614 der Einsatz von niedrig ethoxylierten Fettalkoholen bekannt, mit denen besonders fließfähige und einen guten Perlglanzeffekt aufweisende Konzentrate erhalten werden. Diese Produkte wirken sich jedoch aufgrund ihres Gehaltes an Polyethylenoxid bei bestimmten Anwendungen auf der menschlichen Haut negativ aus. Des weiteren werden in der EP-A-300 379 lineare gesättigte Fettsäuren für ihren Einsatz als Kristallisationshilfsmittel beschrieben. Ebenso ist die Verwendung von Sorbitanfettsäureestern bekannt. Die damit hergestellten Perlglanzkonzentrate sind meist dunkel gefärbt und beeinflussen damit die Farbe der Zubereitung, in die das Perlglanzkonzentrat eingearbeitet ist, negativ.

Als weitere Zusätze können die Perlglanzkonzentrate noch Säuren und/oder Basen sowie Salze zur pH-Wert-Einstellung und Konservierungsmittel enthalten. Zur Einstellung der Viskosität können verschiedene Lösungsmittel, wie z. B. niedermolekulare mehrwertige Alkohole, zugefügt werden.

Polyglycerinester sind für ihren Einsatz in kosmetischen Mitteln bekannt. So werden sie z. B. als Rückfettungsmittel in Haut- und Haarreinigungsformulierungen eingesetzt. Sie sind dermatologisch unbedenklich und belasten, da sie biologisch abbaubar sind, das Abwasser nicht.

Weiterhin werden diese Ester als Emulgatoren, Verdickungs- bzw. Viskositätsregulierungsmittel in vielen kosmetischen Präparaten verwendet.

Die Herstellung der Polyglycerinester und zahlreiche Anwendungsgebiete dieser Polyglycerinester wird bzw. werden z. B. in der DE-A-42 23 407 beschrieben.

Aus der EP-A-0 569 028 ist u. a. die Verwendung von Polyglycerinestern als Viskositätsregulierungsmittel in Shampoos bekannt. Diese Polyglycerinester haben einen hohen Polymerisationsgrad und sollen mindestens sechs Glycerinmonomereinheiten aufweisen. In Formulierungen, die neben diesen Polyglycerinestern als Viskositätsregulierungsmittel auch perlglanzgebende Zusätze enthalten, wird als Kristallisationshilfsmittel zur Perlglanzerzeugung Cocosfettsäuremonoethanolamid zugesetzt.

Aufgabe der Erfindung ist es, Perlglanzkonzentrate zur Verfügung zu stellen, die frei von Fettsäurealkanolamiden und ethoxylierten Fettalkoholen sind und dabei die Eigenschaften der bekannten Perlglanzkonzentrate, wie niedrige Viskosität, gute Lagerstabilität und ausgezeichneten Perlglanzeffekt, besitzen.

Gegenstand der Erfindung sind Perlglanzkonzentrate, die als Kristallisationshilfsmittel ein oder mehrere Polyglycerinester der allgemeinen Formel 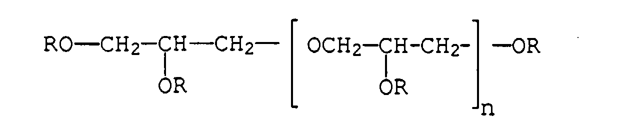 in der
- R: jeweils ein Wasserstoffatom oder einen Acylrest der allgemeinen Formel 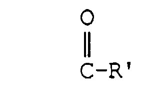 bedeutet, wobei
R' ein Alkylrest mit 9 bis 13 Kohlenstoffatomen ist und
- n =: 1 bis 5 ist,
mit der Maßgabe, daß das molare Verhältnis von R = Wasserstoffatomen zu R = Acylresten 95 : 5 bis 20 : 80 beträgt, enthalten.

Der Veresterungsgrad, d. h. das molare Verhältnis von R = Acylrest zu R = Wasserstoff, sollte dabei so gewählt werden, daß neben veresterten Gruppen immer ein Teil unveresterter Hydroxylgruppen vorhanden ist.

Dadurch werden Perlglanzkonzentrate mit einem brillanten Perlglanz erhalten, die außerordentlich fließfähig sind und dabei in Tensidformulierungen einen Perlglanz hoher optischer Dichte und hervorragender Brillanz erzeugen.

Bevorzugt als Kristallisationshilfsmittel werden dabei Polyglycerinester in Mengen von 1 bis 8 Gew.-%, bezogen auf Dispersionen, eingesetzt.

Als besonders bevorzugt haben sich Polyglycerinester gezeigt, in denen der Rest R ein Caprinoyl-, Lauroyl- oder Myristoylrest ist.

Die erfindungsgemäßen Perlglanzkonzentrate können wie folgt hergestellt werden:

In einem V2A-Kessel mit Rührwerk wird Wasser vorgelegt und auf 80 °C erhitzt. Temperaturindifferente Zusatzstoffe, wie z. B. Citronensäure zur pH-Wert-Einstellung, Dispersionsmittel und Kristallisationshilfsmittel, werden in das heiße Wasser eingerührt, wobei die Mischung auf 80 °C gehalten wird. Die perlglanzgebende Komponente wird diesem Gemisch in auf 80 °C erhitzter Form zugegeben und homogen eingearbeitet. Danach läßt man die Mischung langsam abkühlen. Die langsame Abkühlung des Gemisches ist deshalb zweckmäßig, da so ein gleichmäßiger Perlglanz erzielt wird. Wird die Temperatur von 30 °C unterschritten, können noch temperaturempfindliche Zusatzstoffe, wie z. B. Konservierungsmittel, zugegeben werden.

Die erfindungsgemäßen Perlglanzkonzentrate können bei Raumtemperatur flüssigen Haar- und Körperreinigungsmitteln, flüssigen Geschirrspülmitteln sowie flüssigen Wasch- und Reinigungsmitteln zugesetzt werden. Hierdurch werden Endprodukte erhalten, die einen ausgezeichneten Perlglanz aufweisen. Die hierzu benötigte Menge der Perlglanzdispersion liegt zwischen 1 und 15, bevorzugt 2 bis 5 Gew.-%. Da die erfindungsgemäße Perlglanzdispersion bei Temperaturen über 5 °C eine vergleichsweise niedrige Viskosität aufweist, besteht die Möglichkeit, die Dispersion mit Hilfe von automatischen Pump-, Dosier- und Mischanlagen zu verarbeiten. Von besonderem Interesse ist dies bei der vollkontinuierlichen Herstellung von perlglanzhaltigen Fertigprodukten.

Die erfindungsgemäßen Perlglanzkonzentrate werden an folgenden Beispielen näher erläutert, die Mengenangaben beziehen sich jeweils auf Gew.-%. Die Viskosität wurde mit einem Brookfield-Viskosimeter, Typ RVT bei 6 UpM bei 20 °C, bestimmt.

### Beispiel 1

| Perlglanzkonzentrat | |
|---|---|
| Ethylenglycoldistearat | 20 % |
| Cocosfettsäureamidopropylbetain 30 %ig | 25 % |
| Tetraglycerindilaurat | 4 % |
| Natriumbenzoat | 0,5 % |
| Citronensäuremonohydrat zur pH-Wert-Einstellung (pH 5) | ca. 0,1 % |
| Wasser | |
| | ad. 100 % |
| Viskosität | 14000 mPas |

### Beispiel 2

| Perlglanzkonzentrat | |
|---|---|
| Ethylenglycoldistearat | 12 % |
| Natrium-α-Olefinsulfonat 40 %ig | 40 % |
| Cocosfettsäureamidopropylbetain 30 %ig | 10 % |
| Diglycerinmonolaurat | 3 % |
| Natriumbenzoat | 0,5 % |
| Citronensäuremonohydrat zur pH-Wert-Einstellung (pH 5) | ca. 0,2 % |
| Wasser | |
| | ad. 100 % |
| Viskosität | 8000 mPas |

### Beispiel 3

| Perlglanzkonzentrat | |
|---|---|
| Ethylenglycoldistearat | 10 % |
| Triglycerindilaurat | 5 % |
| Natriumbenzoat | 0,5 % |
| Citronensäuremonohydrat zur pH-Wert-Einstellung (pH 5) | ca. 0,2 % |
| Wasser | |
| | ad. 100 % |
| Viskosität | 6000 mPas |

Diese Perlglanzkonzentrate gemäß den Beispielen 1 bis 3 wurden nach folgenden Kriterien geprüft:
1. Beurteilung des Perlglanzeffektes
2. Lagerstabilität bei höheren und tieferen Temperaturen als Raumtemperatur

Nachstehend sind die entsprechenden Prüfmethoden und Ergebnisse aufgeführt.

### 1. Perlglanzeffekt

Die Beurteilung des Perlglanzeffektes erfolgte visuell nach den Einstufungen matt bis brillant. Es wurden zunächst die Perlglanzkonzentrate entsprechend den Beispielen 1 bis 3 direkt beurteilt und anschließend 2 gew.-%ige Verdünnungen in entsalztem Wasser hergestellt.

Die erfindungsgemäßen Perlglanzkonzentrate besitzen einen ausgeprägten Perlglanzeffekt, der sich in einer hohen Brillanz ausdrückt.

### 2. Lagerstabilität bei höheren und tieferen Temperaturen

Die genannten Perlglanzkonzentrate, entsprechend den Beispielen 1 bis 3, wurden zehn Gefrier-/Tau-Zyklen von +25 °C auf -5 °C unterworfen und vier Monate bei +40 °C gelagert. Nach Beendigung dieser Belastungsversuche konnten keine Anzeichen von Instabilität, z. B. Sedimentationen, festgestellt werden.

Die erfindungsgemäßen Perlglanzdispersionen zeigen nach den vorgenannten Prüfungskriterien, daß sie die Anforderungen an Perlglanzkonzentrate des Standes der Technik nicht nur erfüllen, sondern sogar übertreffen, da sie keine Fettsäurealkanolamide und/oder ethoxylierte Fettalkohole enthalten und deshalb frei von sonst gegebenenfalls entstehenden schädlichen Abbauprodukten sind.

### Anwendungsbeispiel

| Haarshampoo | |
|---|---|
| Natriumlaurylethersulfat (28 %ig) | 40 % |
| Cocosfettsäureamidopropylbetain (37,5 %ig) | 10 % |
| Natriumchlorid | 0,5 % |
| Perlglanzkonzentrat Beispiel 3 | 5 % |
| Wasser | |
| | ad. 100 % |
| Viskosität | 2000 mPas |

Dieses Shampoo weist einen brillanten Perlglanz auf und kann über lange Zeit, ohne instabil zu werden, gelagert werden.

## Patentansprüche

1. Wäßrige, fließfähige Perlglanzkonzentrate, welche frei von Fettsäurealkanolamiden und ethoxylierten Fettalkoholen sind und
a) 10 - 40 % perlglanzgebende Komponenten,
b) 0 - 50 % Dispergierhilfsmittel und
c) 0,2 - 20 % Kristallisationshilfsmittel
enthalten, dadurch gekennzeichnet, daß als Kristallisationshilfsmittel ein oder mehrere Polyglycerinester der allgemeinen Formel 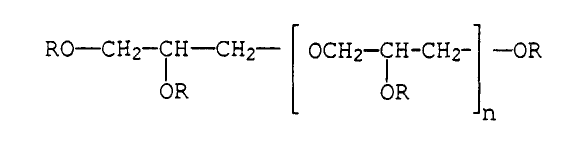 in der
R jeweils ein Wasserstoffatom oder einen Acylrest der allgemeinen Formel 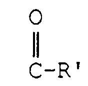 bedeutet, wobei
R' ein Alkylrest mit 9 bis 13 Kohlenstoffatomen ist und
n = 1 bis 5 ist,
mit der Maßgabe, daß das molare Verhältnis von R = Wasserstoffatomen zu R = Acylresten 95 : 5 bis 20 : 80 beträgt, enthalten sind.

## Claims

1. Aqueous, free-flowing pearly lustre concentrates which are free from fatty acid alkanolamides and ethoxylated fatty alcohols and comprise
a) 10 - 40% of pearlizing components,
b) 0 - 50% of dispersion auxiliaries and
c) 0.2-20% of crystallization auxiliaries,
characterized in that the crystallization auxiliaries present are one or more polyglycerol esters of the general formula 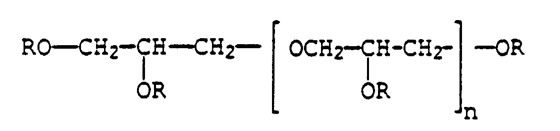 in which
R is in each case a hydrogen atom or an acyl radical of the general formula 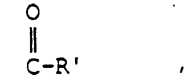 where
R' is an alkyl radical having from 9 to 13 carbon atoms, and
n = 1 to 5,
with the proviso that the molar ratio of R = hydrogen atoms to R = acyl radicals is from 95 : 5 to 20 : 80.

## Revendications

1. Produits concentrés à lustre nacré, aqueux et coulants, qui sont exempts d'alcanolamides d'acide gras et d'alcools gras éthoxylés et qui contiennent
a) 10-40% de composants donnant un lustre nacré,
b) 0-50% d'un agent auxiliaire de dispersion, et
c) 0,2-20% d'un agent auxiliaire de cristallisation,
caractérisés en ce que sont contenus comme agent auxiliaire de cristallisation, un ou plusieurs poly(esters de glycérol) de formule générale 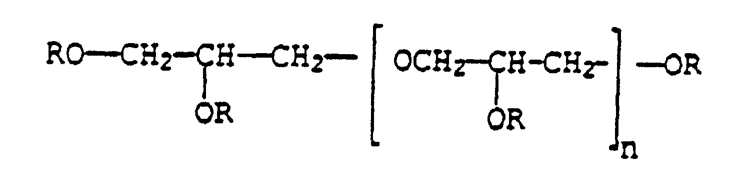 dans laquelle
R représente dans chaque cas un atome d'hydrogène ou un radical acyle de formule générale 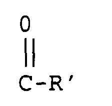 dans laquelle
R' est un radical alkyle ayant 9 à 13 atomes de carbone et n est égal à 1 à 5,
à condition que le rapport molaire de R = atomes d'hydrogène à R = radicaux acyle soit 95:5 à 20:80.
